# EUROPEAN PATENT APPLICATION

(11) **EP 4 834 384 A1**
(43) Date of publication of application: **04.11.2026**
(21) Application number: 25173631.0
(22) Date of filing: 30.04.2025
(51) Int. Cl.: A61M 1/06, A61M 1/00

(54) **A METHOD FOR REGULATING THE OPERATION OF A MEDICAL PUMP AND SUCH A MEDICAL PUMP**

(71) Applicant: CARAG AG, 6340 Baar (CH)
(72) Inventor: Larsson, Michael, 6302 Zug (CH); Bernhard, Jérôme, 8003 Zürich (CH)
(74) Representative: Grünecker Patent- und Rechtsanwälte PartG mbB

(57) **Abstract**

The present invention relates to a method for regulating the operation of a medical pump, such as a breast pump, for applying a negative pressure to a suction side of said medical pump, wherein the medical pump comprises an aggregate for applying a negative pressure to a suction side of said medical pump to generate a fluid flow from said suction side to a collection side of the medical pump, wherein said aggregate is operatively connected to a controller for controlling the operation of the medical pump, wherein the controller is operatively connected to a user interface, wherein input over the user interface instructs the controller to adjust at least one parameter of a negative pressure profile over time, characterized in that said parameter is a pressure gradient. The present invention also relates to a medical pump, such as a breast pump, comprising an aggregate for applying a negative pressure to a suction side of said medical pump to generate a fluid flow from said suction side to a collection side of the medical pump, a controller operatively connected to said aggregate and provided for controlling the operation of the medical pump, a user interface operatively connected to the controller, wherein the user interface is provided for the controller to adjust at least one parameter of a negative pressure profile over time, characterized in that said parameter is a pressure gradient.

## Description

The present invention relates to a method for regulating the operation of a medical pump for applying a negative pressure to a suction side of said medical pump and further relates to such a medical pump.

Medical pumps that apply a negative pressure, also known as suction or vacuum pumps, find their implementation in various medical fields and applications and are generally administered to patients to establish a fluid flow away from said patient. Such medical pumps are commonly realized as breast pumps for the extraction of breastmilk from the patient, drainage pumps for the removal of clinically undesirable fluids, such as air or bodily fluids, or medical pumps for use in negative pressure wound therapy. In the last use, the fluid flow established by the application of a negative pressure from the medical pump not only enables the removal of wound exudate from the application site but may also provide vacuum stimulation to facilitate the healing process thereof. As such, medical pumps are typically configured and adapted to provide a negative pressure directly to the patient's or user's body.

The direct application of negative pressure to a bodily area, particularly one that is not in a natural condition but is suffering from exceptional circumstances, such as soreness, sensitivity, or wounding, may evoke new and further unpleasant sensations for the user. The provocation of pain during use of such a medical pump may result in a user's refusal to complete corresponding treatments or therapies, ultimately generating a vicious circle of pain and non-healing. However, the definition and classification of pain cannot be universally identified but is rather an individually based variable that includes such factors as tolerance levels, the application location of the negative pressure, i.e. where on the body the vacuum is applied, the application duration, as well as past pain memories from negative experiences. Therefore, the application of negative pressure via a medical pump should be set and adjusted with the patient's or user's perception in mind.

It is generally well known that the absolute negative pressure level applied to a user or patient may be directly responsible for the pain and discomfort felt during implementation of a medical pump. As such, certain medical pumps may allow the user to reduce the negative pressure level or the duration thereof during use, in an attempt to reduce acute pain responses while still allowing for continued use of the medical pump. For example, a breast pump according to US 2021/0060220 A1 comprises a user interface, through which the user can change certain pump settings, in order to optimize their pumping experience during application of the cyclically changing negative pressure values. Specifically, the user can influence the negative pressure level, amplitude, frequency, speed and waveform of the applied negative pressure, all parameters that relate to the absolute negative pressure level values applied to the user.

Although the adjustment of certain pumping settings and parameters may alleviate the user's discomfort during use of the medical pump, it may simultaneously reduce the overall efficiency of the medical pump, resulting in longer application durations, which in turn may re-result in further unpleasantness for the user or patient.

Therefore, the present invention aims to provide a method for regulating the operation of a medical pump, in addition to such a corresponding medical pump, which may not only improve the user's satisfaction during use but may also maintain the generally optimized pumping parameters thereof.

As a solution thereto, the present invention provides a method, as defined in claim 1, and a corresponding medical pump, as defined in claim 7.

According to the first aspect of the present invention, there is provided a method for regulating the operation of a medical pump for applying a negative pressure to a suction side of said medical pump, wherein the medical pump comprises an aggregate for applying a negative pressure to a suction side of said medical pump to generate a fluid flow from said suction side to a collection side of the medical pump, wherein said aggregate is operatively connected to a controller for controlling the operation of said medical pump and wherein the controller is operatively connected to a user interface. Input received over the user interface instructs the controller to adjust at least one parameter of a negative pressure profile over time, specifically, a pressure gradient.

A medical pump is preferably any means that can generate a negative pressure on a suction side of said medical pump, thereby enabling the transport of fluid from said suction side to a collection side of the medical pump. In order to do so, the medical pump comprises an aggregate that is preferably operatively connected to a motor. In particular, the aggregate may further comprise at least one or more of a membrane and piston component.

The fluid conveyed from the suction side to the collection side of the medical pump may include at least one or more of a gaseous medium, such as air, a liquid, for example breastmilk or wound exudate, and solid particulates, such as coagulated blood or breastmilk. Preferably, the collection side of the medical pump is provided with a reservoir for collecting the fluid. Moreover, the suction side of the medical pump is preferably provided with a negative pressure attachment, which may enable the generated negative pressure to reach its application location, for example the user's body. A negative pressure attachment may include such components including at least one or more of a breast shield, a tubing system and a film seal.

In a preferred embodiment, the medical pump is a breast pump, a drainage pump, or a pump for use in negative pressure wound therapy. It is understood that the term breast pump includes breast pumps for both portable and pumping-station applications, the former of which may also include in-bra breast pumps.

The controller of the medical pump may be operatively connected to the aggregate by means of a wireless or wired connection.

The controller of the medical pump may be operatively connected to the user interface by means of a wireless or wired connection.

The user interface is adapted to receive input, preferably from the user, and provide said input to the controller, in order to regulate the operation of the medical pump. More specifically, the input provided over the user interface instructs the controller to adjust at least one parameter of the negative pressure profile over time. The controller may adjust such a parameter by regulating the aggregate so that the negative pressure profile over time generated by the medical pump and applied to the user reflects the input provided. By enabling the user to directly provide their personal feedback and sensibilities to the user interface and having these result in the adjustment of the medical pump operation, the comfort and ease of use for the user can be increased, and consequently the pain during use decreased.

In a preferred embodiment, the user interface may be a touch screen, a setting dial or at least one press button, more preferably at least two press buttons. Preferably, the medical pump further comprises a housing, on which the user interface is located.

Preferably, the user interface is adapted to allow the user to provide input during operation of the medical pump so that the pumping parameters are adjusted during use. The user may also set the pumping parameters prior to operation of the medical pump.

In an alternative or additional embodiment, the user interface may be accessed via a mobile device, in particular via a software application, such as web or mobile application.

The negative pressure profile over time is the curve progression of the negative pressure generated by the medical pump and applied to suction side thereof, e.g. to the user. Consequently, various parameters may be used to define said curve progression and therefore adjust the pressure generated on the suction side of the medical pump. Such parameters include but are not limited to vacuum or negative pressure level, amplitude, waveform, frequency, and pressure gradient.

As contingent on technical aspects of the medical pump, during initial operation of said medical pump, the negative pressure profile over time may begin at a pressure associated with an ambient atmosphere pressure. The aggregate of the medical pump may then be regulated to apply a negative pressure to the suction side of medical pump. This negative pressure may be held constant over a certain period of time and may be designated as a vacuum or negative pressure level, which is associated with an absolute negative pressure value. The section of the negative pressure profile over time between the initial atmospheric pressure value and the designated negative pressure level is known as a vacuum build-up segment, since an increase in vacuum is generated during this time.

In a preferred embodiment of the invention, the negative pressure of the medical pump alternates between different negative pressure levels. Said negative pressure levels may include at least one or more of the following pressures: ambient atmospheric pressure and any vacuum values between 0 and -300 mmHg.

The difference between the negative pressure levels is known as an amplitude of the negative pressure profile over time and is considered a relative value. Preferably, the amplitude is between 50 and 250 mmHg and more preferably between 100 and 200 mmHg.

The waveform of a curve progression is the shape of its graph as a function of time, independent of its magnitude and time scales, as well as any displacement in time. The waveform of the negative pressure profile over time may remain constant during operation of the medical pump; however, the negative pressure profile over time may exhibit multiple, preferably at least two, more preferably at least three, different waveforms during operation. Preferably, at least one waveform of the negative pressure profile over time is periodic or cyclic and repeats itself regularly at a constant period. Examples of commonly implemented periodic or cyclic waveforms may include a sine waveform, square waveform, triangle waveform and a sawtooth waveform; however, composite waveforms combining different waveforms and curve progressions may also be implemented in a periodic fashion.

Preferably, the negative pressure profile over time exhibits at least a stimulation stage and an expression stage. The stimulation stage may be defined by waveform with a higher frequency and lower amplitude in comparison to a lower frequency and higher amplitude of the waveform of the expression stage. Furthermore, the negative pressure profile over time may further include a transition stage between the stimulation stage and the expression stage. The transition stage may be exhibit amplitudes and frequencies between those of the stimulation and expression stages.

Preferably, at least one waveform of the negative pressure profile over time is divided into a vacuum build-up segment, during which the negative pressure increases, and a vacuum release segment, wherein the negative pressure increases. Between the vacuum build-up segment and the vacuum release segment, the negative pressure level may be held constant.

According to the invention, at least the pressure gradient of the negative pressure profile over time is adjusted via input over the user interface. The pressure gradient is the rate at which the negative pressure changes over time.

Surprisingly, pressure gradient has not only been shown to have a direct correlation with a user's pain perception, i.e. that certain pressure gradients inflict more discomfort than others, but also has an indirect influence on the user's overall sensitivity and reactivity to other pumping parameters. With respect to the latter, it may be possible to increase a user's pain tolerance to a specific negative pressure level by implementing a reduced pressure gradient when reaching said negative pressure level. Reciprocally, if a pressure gradient is adjusted to higher values not only may the user feel discomfort during application of the changing negative pressure but may not be able to tolerate the targeted negative pressure value, consequently desiring the adjustment of multiple pumping parameters. This may impact the efficiency and optimization of the medical pump and overall reduce the user's satisfaction therewith.

Although the pressure gradient may be constant, it has been shown that varying the pressure gradient may improve the user's reception to discomfort and pain. Preferably, a vacuum build-up segment of the negative pressure profile over time may exhibit at least two, preferably at least three, different pressure gradients. Furthermore, a first pressure gradient of the vacuum build-up segment of the negative pressure profile over time may be lower than that of a following pressure gradient.

In a preferred embodiment, the pressure gradient of a vacuum build-up segment of the at least one waveform is adjusted by the controller. Furthermore, an amplitude and a frequency parameter may further define said waveform. Preferably, at least one or more of the amplitude and frequency parameters may be provided as pre-set values provided by the medical pump. Furthermore, at least one or more of the amplitude and frequency parameters may be also provided as input via the user interface. At least one or more of the amplitude and frequency parameters may also be adjusted during operation of the medical pump and therefore during use of said medical pump. The controller may adjust the pressure gradient of said waveform while maintaining the defined amplitude, the defined frequency, or preferably, both the defined amplitude and defined frequency. Preferably, the amplitude is maintained. These parameters may be maintained by adjusting the pressure gradient of the corresponding vacuum release segment of the waveform.

In a preferred embodiment, the pressure gradient is adjusted to a value between 100 and 1500 mmHg/sec, preferably between 300 and 1000 mmHg/sec, more preferably between 400 and 800 mmHg/sec and most preferably between 400 and 500 mmHg/sec.

According to the second aspect of the present invention, there is provided medical pump, such as a breast pump, comprising an aggregate for applying a negative pressure to a suction side of said medical pump to generate a fluid flow from said suction side to a collection side of the medical pump, a controller operatively connected to said aggregate and provided for controlling the operation of the medical pump, and a user interface operatively connected to the controller, wherein the user interface is provided for the controller to adjust at least one parameter of a negative pressure profile over time, specifically, a pressure gradient.

In a preferred embodiment, the medical pump further comprises a data storage medium, whereby the data storage medium includes at least one pre-determined value of the pressure gradient and whereby the user interface is operatively connected to the data storage medium to select the pre-determined value. Preferably, the data storage medium includes at least three pre-determined pressure gradient values, including a minimum value, a maximum value and an increment value.

The maximum pre-determined pressure gradient may be corrected to a value known to generally cause discomfort in the user. Such a maximum value may not exceed 1500 mmHg/sec, preferably 1000 mmHg/sec, more preferably 800 mmHg/sec, and most preferably 500 mmHg/sec. The minimum pre-determined pressure gradient may relate to a lower limit, below which the medical pump is unable to efficiently provide the required fluid flow from the suction side thereof. Such a minimum value is preferably 100 mmHg/sec, more preferably 300 mmHg/sec, and most preferably 400 mmHg/sec. The increment value may correspond to the interval by which the pressure gradient may be adjusted. The increment value is preferably 5 mmHg/sec, more preferably 10 mmHg/sec, and most preferably 25 mmHg/sec.

Preferably, the data storage medium is adapted to record the input provided by the user interface.

The data storage medium may be operatively connected to a processor that is configured to provide at least one or more of real-time feedback and individual recommendations of at least one parameter of the negative pressure profile over time, preferably the pressure gradient. The processor may also provide an individual recommendation of a combination of a pressure gradient and a negative pressure level. Such a recommendation may include a lower pressure gradient with an increased negative pressure level. This may induce a higher pain tolerance in the user and consequently increased medical pump optimization. Alternatively, since pain perception is individually perceived, the recommendation of the data storage medium may also recommend a higher pressure gradient with an increased negative pressure level.

Preferably, the processor is adapted to access and evaluate the input recorded on the data storage medium, in order to provide real-time feedback and individual recommendation. The processor may be adapted to run a data-driven model to at least analyze, calculate, and/or suggest user-specific recommendations with respect to at least one parameter of the parameters of a negative pressure profile over time. With respect to an individual recommendation, the processor may evaluate various input data and values provided through the user interface. By obtaining more data points over time, the processor may be able to assess which parameters of a negative pressure profile over time a user has generally implemented, which parameters of a negative pressure profile over time the user has avoided, and which parameters of a negative pressure profile over time the user has preferred, for example. The processor may also be able to associate the parameters of a negative pressure profile over time input by the user to points on a pain perception scale, thereby establishing a relationship between different values of the parameters of a negative pressure profile over time and the user's discomfort level. By evaluating and analyzing said information, the processor may be configured to learn from the input provided via the user interface and provide individualized recommendations for current and future pumping parameters for said user based thereupon. Such individual recommendations may be utilized for future medical pumps.

Furthermore, the processor may be operatively connected to a resource pool, such as a cloud, and adapted to compare the input from the user interface and the input from other medical pumps. Such a comparison may be included in the individual recommendations provided by the processor. The resource pool may include at least one parameter of the negative pressure profile over time, preferably the pressure gradient, for a different medical pump. The processor may be adapted to compare the input of the user interface with the at least one parameter of the negative pressure profile over time of the resource pool. Furthermore, the processor may also be adapted to compare the input of the user interface with at least two or more of the parameters, preferably all of the parameters, of the negative pressure profile over time of the resource pool, for example the negative pressure level, the frequency, and the amplitude, amongst others.

The processor may be further configured to access both input recorded on the data storage medium and parameter values stored in a resource pool. The processor may also be further configured to access data from the resource pool and implement such when providing individual recommendations to the user. Preferably, the processor may also provide individual recommendations to the resource pool for use for future medical pumps.

The operative connection between the data storage medium and the processor may be wired or wireless. The operative connection between the processor and the resource pool is preferably wireless.

Preferably, the user interface further comprises a display. The display may be adapted to display at least one or more of the pre-determined value of the pressure gradient, input provided to the user interface, input recorded on the data storage medium, real-time feedback, individual recommendations, and further pumping parameters.

Preferably, the controller of the medical pump is adapted to adjust the pressure gradient of the vacuum build-up segment and to maintain the amplitude, the frequency, or both the amplitude and frequency defining at least one waveform of the negative pressure profile over time.

In a further aspect of the inventive medical pump, the aggregate of the medical pump may further comprise a motor, wherein the speed of the motor is regulated by the controller and wherein the controller is adapted to adjust the speed of the motor to achieve the pressure gradient of the negative pressure profile over time. In particular, the motor may be self-commutated, with a mechanical commutator (AC, DC) or electronic commutator (AC), for example, or externally commutated, either asynchronous (AC) or synchronous (AC).Furthermore, the aggregate may further comprise at least one or more of a membrane and piston component.

According to a preferred embodiment, the medical pump may further comprise a leakage adjustment means that is operatively connected to the controller and adapted to allow air to enter the suction side of the medical pump to achieve the pressure gradient of the negative pressure profile over time. A leakage adjustment means may be realized by a leakage valve. By allowing air to access the suction side of the medical pump, the negative pressure on said side may be reduced, thereby altering the pressure gradient and adjusting it to correspond to the input provided through the use interface.

This invention may also be said broadly to consist in the parts, elements and features referred to or indicated in the specification of the application, individually or collectively, and any or all combinations of any two or more said parts, elements or features, and where specific integers are mentioned herein which have known equivalents in the art to which this invention relates, such known equivalents are deemed to be incorporated herein as if individually set forth. As used herein "(s)" following a noun could mean the plural and/or singular forms of the noun.

Further details, advantages or features of the present invention will become apparent from the following description in combination with the figures. In the figures:
Figure 1 illustrates a schematic drawing of a negative pressure profile over time, and
Figure 2 illustrates a schematic drawing of an alternative negative pressure profile over time.

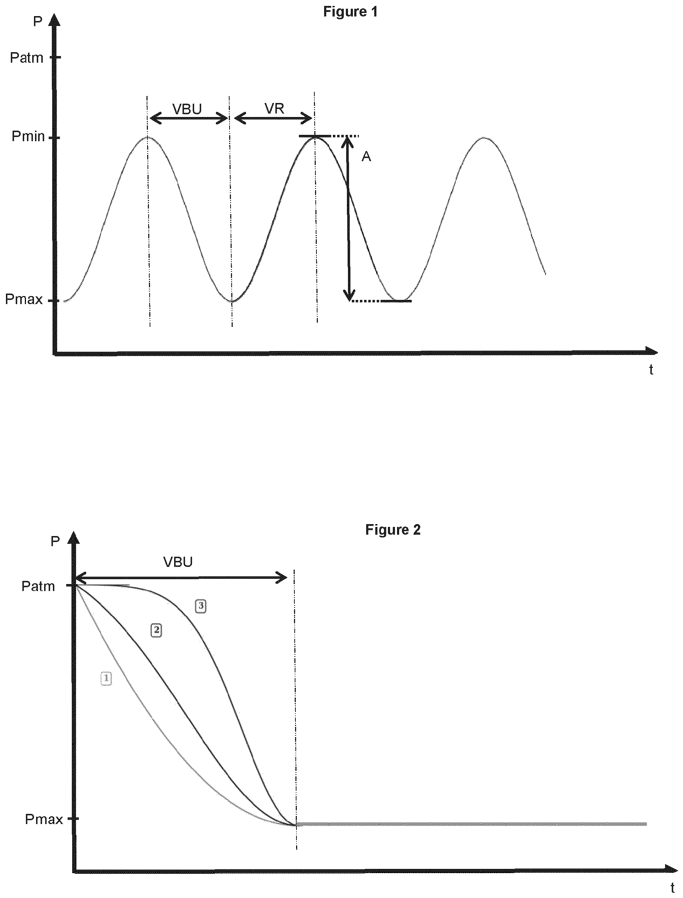

The negative pressure profile over time, as depicted in Figure 1, exhibits a periodic waveform as defined by a sine wave. Said waveform alternates between two negative pressure levels designated as a minimum negative pressure Pmin and a maximum negative pressure Pmax. As given on the y-axis of the graph, which represents the negative pressure, both the minimal negative pressure Pmin and a maximum negative pressure Pmax are below the ambient atmospheric pressure Patm. The difference between the minimum negative pressure Pmin and a maximum negative pressure Pmax is given as the amplitude A. Moreover, the waveform depicted in Figure 1 is divided into a vacuum build-up segment VBU, during which the negative pressure increases, and a vacuum release segment VR, wherein the negative pressure increases.

Figure 2 illustrates an alternative negative pressure profile over time, wherein said curve initiates at the ambient atmospheric pressure Patm and progresses to a constant negative pressure level detonated by the maximum negative pressure Pmax. Moreover, said figure exhibits three different curve progressions between these two points, i.e. during vacuum build-up segment VBU, that represent three different pressure gradients that may be adjusted and set for the medical pump.

### List of Reference Signs

- P: pressure
- Patm: ambient, atmospheric pressure
- Pmin: minimum negative pressure level
- Pmax: maximum negative pressure level
- VBU: vacuum build-up segment
- VR: vacuum release segment
- A: amplitude
- t: time

## Claims

1. A method for regulating the operation of a medical pump, such as a breast pump, for applying a negative pressure to a suction side of said medical pump,
wherein the medical pump comprises an aggregate for applying a negative pressure to a suction side of said medical pump to generate a fluid flow from said suction side to a collection side of the medical pump, wherein said aggregate is operatively connected to a controller for controlling the operation of the medical pump,
wherein the controller is operatively connected to a user interface,
wherein input over the user interface instructs the controller to adjust at least one parameter of a negative pressure profile over time,
**characterized in that** said parameter is a pressure gradient.

2. A method according to claim 1, **characterized in that** the negative pressure profile over time exhibits at least one waveform which is divided into a vacuum build-up segment and a vacuum release segment and wherein the pressure gradient of the vacuum build-up segment is adjusted.

3. A method according to claim 2, **characterized in that** the waveform is further defined by an amplitude value and a frequency value and that the controller maintains at least the ampltiude during adjustment of the pressure gradient of the vacuum build-up segment.

4. A method according to claim 3, **characterized in that** both the amplitude and frequency values are maintained during adjustment of the pressure gradient of the vacuum build-up segment, by adjusting the pressure gradient of the vacuum release segment.

5. A method according to any of the previous claims, **characterized in that** the at least one waveform is a periodic waveform.

6. A method according to any of the previous claims, **characterized in that** the pressure gradient is adjusted to a value between 100 and 1500 mmHg/sec, preferably between 300 and 1000 mmHg/sec, more preferably between 400 and 800 mmHg/sec and most preferably between 400 and 500 mmHg/sec.

7. A medical pump, such as a breast pump, comprising
an aggregate for applying a negative pressure to a suction side of said medical pump to generate a fluid flow from said suction side to a collection side of the medical pump,
a controller operatively connected to said aggregate and provided for controlling the operation of the medical pump,
a user interface operatively connected to the controller, wherein the user interface is provided for the controller to adjust at least one parameter of a negative pressure profile over time,
**characterized in that** said parameter is a pressure gradient.

8. A medical pump according to claim 7, **characterized in that** the medical pump further comprises a data storage medium,
whereby the data storage medium includes at least one pre-determined value of the pressure gradient, preferably three pre-determined values including a minimum value, a maximum value and an increment value,
and whereby the user interface is operatively connected to the data storage medium to select the pre-determined value.

9. A medical pump according to claim 8, **characterized in that** the data storage medium is operatively connected to a processor,
whereby the processor is configured to provide at least one of real-time feedback and individual recommendation for at least one parameter of the negative pressure profile over time, preferably the pressure gradient.

10. A medical pump according to claim 9, **characterized in that** the processor is operatively connected to a resource pool, such as a cloud,
whereby the resource pool includes at least one parameter of the negative pressure profile over time for a different medical pump, preferably the pressure gradient,
and wherein the processor is adapted to compare the input of user interface with the at least one parameter of the negative pressure profile over time of the resource pool, preferably all of the parameters of the negative pressure profile over time of the resource pool .

11. A medical pump according to any of the previous claims, **characterized in that** the negative pressure profile over time exhibits at least one waveform, which is divided into a vacuum build-up segment and a vacuum release segment and is further defined by an amplitude and frequency value,
wherein the controller is adapted to adjust the pressure gradient of the vacuum build-up segment and to maintain the amplitude, the frequency, or both the amplitude and frequency defining said waveform.

12. A medical pump according to any of the previous claims, **characterized in that** the medical pump further comprises a leakage adjustment means, such as a leakage valve, that is operatively connected to the controller and adapted to allow air to enter the suction side of the medical pump to achieve the pressure gradient of the negative pressure profile over time.

13. A medical pump according to any of the previous claims, **characterized in that** the aggregate of the medical pump further comprises a motor
wherein the speed of the motor is regulated by the controller, and
wherein the controller is adapted to adjust the speed of the motor to achieve the pressure gradient of the negative pressure profile over time.

14. A medical pump according to any previous claims, **characterized in that** the negative pressure profile over time exhibits at least a stimulation stage and an expression stage, preferably also a transition stage between the stimulation stage and the expression stage.

15. A medical pump according to any previous claims, **characterized in that** the waveform of the stimulation stage differs from the waveform of the expression stage.
